# EUROPÄISCHE PATENTSCHRIFT

(11) **EP 0 456 089 B1**
(45) Veröffentlichungstag und Bekanntmachung des Hinweises auf die Patenterteilung: **03.04.1996**
(21) Anmeldenummer: 91106992.0
(22) Anmeldetag: 30.04.1991
(51) Int. Cl.: C07D 309/06, C07D 335/02, C07D 307/14, C07D 317/28, C07D 213/24, C07C 251/52, A01N 43/02, A01N 43/08, A01N 43/16, A01N 43/18, A01N 43/40

(54) **Cyclohexenonoximether, Verfahren zu ihrer Herstellung und ihre Verwendung als Herbizid**
Cyclohexenonoximethers, process for their production and their application as herbicides
Ethers de cyclohexénonoximes, leur procédé de production et leur application comme herbicides

(30) Priorität: 09.05.1990 DE 4014988
(43) Veröffentlichungstag der Anmeldung: 13.11.1991
(73) Patentinhaber: BASF Aktiengesellschaft, D-67063 Ludwigshafen (DE)
(72) Erfinder: Kast, Juergen, Dr., W-6737 Boehl-Iggelheim (DE); Meyer, Norbert, Dr., W-6802 Ladenburg (DE); Misslitz, Ulf, Dr., W-6730 Neustadt (DE); Harreus, Albrecht, Dr., W-6700 Ludwigshafen (DE); Walter, Helmut, Dr., W-6719 Obrigheim (DE); Gerber, Matthias, Dr., W-6704 Mutterstadt (DE); Westphalen, Karl-Otto, Dr., W-6720 Speyer (DE)

(56) Entgegenhaltungen:
- EP-A- 0 131 875
- EP-A- 0 133 349
- EP-A- 0 243 313
- EP-A- 0 368 227
- DE-A- 3 838 309

## Beschreibung

Die Erfindung betrifft neue herbizid wirksame Cyclohexenonoximether der Formel I 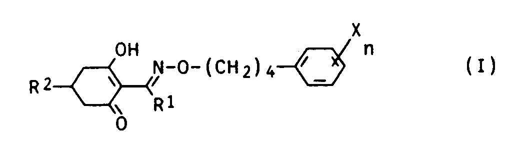
in der die Variablen folgende Bedeutung haben:
- R¹: eine C₁-C₆-Alkylgruppe;
- X: Halogen
- n: 1 bis 5;
- R²: eine C₁-C₄-Alkoxy-C₁-C₆-alkyl- oder C₁-C₄-Alkylthio-C₁-C₆-alkylgruppe;
eine C₃-C₇-Cycloalkylgruppe oder eine C₅-C₇-Cycloalkenylgruppe, wobei diese Gruppen noch einen bis drei Reste tragen können, ausgewählt aus einer Gruppe, bestehend aus C₁-C₄-Alkyl, C₁-C₄-Alkoxy, C₁-C₄-Alkylthio, C₁-C₄-Halogenalkyl, Hydroxy und Halogen;
ein 5-gliedriger gesättigter Heterocyclus der ein oder zwei Heteroatome, ausgewählt aus einer Gruppe bestehend aus Sauerstoff und Schwefel, enthält und der noch einen bis drei Reste tragen kann, ausgewählt aus einer Gruppe, bestehend aus C₁-C₄-Alkyl, C₁-C₄-Alkoxy, C₁-C₄-Alkylthio und C₁-C₄-Halogenalkyl;
ein 6- oder 7-gliedriger gesättigter, ein- oder zweifach ungesättigter Heterocyclus, enthaltend ein oder zwei Heteroatome, ausgewählt aus einer Gruppe bestehend aus Sauerstoff und Schwefel, wobei der Heterocyclus noch einen bis drei Reste tragen kann, ausgewählt aus einer Gruppe, bestehend aus Hydroxy, Halogen, C₁-C₄-Alkyl, C₁-C₄-Alkoxy, C₁-C₄-Alkylthio und C₁-C₄-Halogenalkyl;
ein 5-gliedriger Heteroaromat, enthaltend ein bis drei Heteroatome, ausgewählt aus einer Gruppe bestehend aus zwei Stickstoffatomen und einem Sauerstoff- oder Schwefelatom, wobei dieser Ring noch einen bis drei Reste tragen kann, ausgewählt aus einer Gruppe, bestehend aus Halogen, Cyano, C₁-C₄-Alkyl, C₁-C₄-Alkoxy, C₁-C₄-Alkylthio, C₁-C₄-Halogenalkyl, C₂-C₆-Alkenyl, C₂-C₆-Alkenyloxy, C₂-C₆-Halogenalkenyl und C₁-C₄-Alkoxy-C₁-C₄-alkyl;
die Phenylgruppe oder die Pyridylgruppe, wobei diese Gruppen noch einen bis drei Reste tragen können, ausgewählt aus einer Gruppe, bestehend aus Halogen, Nitro, Cyano, C₁-C₄-Alkyl, C₁-C₄-Alkoxy, C₁-C₄- Alkylthio, C₁-C₄-Halogenalkyl, C₃-C₆-Alkenyloxy, C₃-C₆-Alkinyloxy und -NR³R⁴, worin
- R³: Wasserstoff, eine C₁-C₄-Alkylgruppe, eine C₃-C₆-Alkenylgruppe oder eine C₃-C₆-Alkinylgruppe und
- R⁴: Wasserstoff, eine C₁-C₄-Alkylgruppe, eine C₃-C₆-Alkenylgruppe, eine C₃-C₆-Alkinylgruppe, eine C₁-C₆-Acylgruppe oder ein Benzoylrest, wobei der aromatische Ring noch einen bis drei Reste tragen kann, ausgewählt aus einer Gruppe, bestehendaus Nitro, Cyano, Halogen, C₁-C₄-Alkyl, C₁-C₄-Alkoxy, C₁-C₄-Alkylthio und C₁-C₄-Halogenalkyl
bedeuten,
sowie die landwirtschaftlich nutzbaren Salze von I und die Ester von I mit C₁-C₁₀-Carbonsäuren oder anorganischen Säuren.

Außerdem betrifft die Erfindung ein Verfahren zu ihrer Herstellung sowie ihre Anwendung als Pflanzenschutzmittel.

Die erfindungsgemäßen Cyclohexenonoximether I haben offensichtlich sauren Charakter, d.h. sie können einfache Umsetzungsprodukte wie Salze von Alkali- oder Erdalkaliverbindungen oder Enolester bilden.

Die Verbindungen der Formel 1 können in mehreren tautomeren Formen auftreten, die alle vom Anspruch erfaßt werden.

In der DE-A 38 38 309 und der korrespondierenden europäischen Anmeldung EP-A-0 368 227 sind herbizid wirksame Cyclohexenonoximether beschrieben, deren allgemeine Formel die eingangs definierten Arylbutylenoximinocyclohexandione I umfaßt. Speziell aufgeführt sind u.a.
2-{1-[4-(3-Fluorphenyl)-butyloximino]-propyl}-5-(2-ethylthio-propyl)-3-hydroxy-cyclohex-2-enon,
2-{1-[4-(3-Fluorphenyl)-butyloximino]-butyl}-5-(2-ethylthio-propyl)-3-hydroxy-cyclohex-2-enon,
2-{1-[4-(4-Fluorphenyl)-butyloximino]-propyl}-5-(2-ethylthio-propyl)-3-hydroxy-cyclohex-2-enon und
2-{1-[4-(4-Fluorphenyl)-butyloximino]-butyl}-5-(2-ethylthio-propyl)-3-hydroxy-cyclohex-2-enon.

Der Erfindung lag die Aufgabe zugrunde, Cyclohexenonoximether zu synthetisieren, die gegenüber den bekannten Vertretern dieser Stoffklasse eine größer Selektivität, bei der Bekämpfung von Ungräsern im grasartigen Kulturen wie z.B. Reis, aufweisen.

Entsprechend dieser Aufgabe wurden die eingangs definierten neuen Cyclohexenonoximether der Formel I gefunden, die eine gute herbizide Wirkung vorzugsweise gegen Arten aus der Familie der Gräser (Gramineen) besitzen. Sie sind verträglich und somit selektiv in breitblättrigen Kulturen sowie in monokotylen Gewächsen, welche nicht zu den Gramineen zählen. Ferner sind Verbindungen darunter, welche sich auch in Gramineenkulturen selektiv verhalten und geleichzeitig unerwünschte Gräser bekämpfen.

Die Cyclohexenone der Formel I können in an sich bekannter Weise aus schon bekannten Derivaten der Formel II (EP-A 80 301, EP-A-125 094, EP-A 142 741, US-A 4 249 937, EP-A 137 174 und EP-A 177 913) und den entsprechenden Hydroxylaminen der Formel III hergestellt werden (EP-A 169 521).

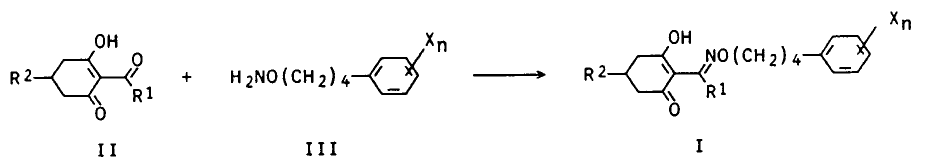

Zweckmäßig führt man die Umsetzung in heterogener Phase in einem Lösungsmittel, bei einer ausreichenden Temperatur unterhalb von etwa 80°C, in Gegenwart einer Base durch und verwendet das Hydroxylamin III in Form seines Ammoniumsalzes.

Geeignete Basen sind z. B. Carbonate, Hydrogencarbonate, Acetate, Alkoholate oder Oxide von Alkali- oder Erdalkalimetallen, insbesondere Natriumhydroxid, Kaliumhydroxid, Magnesiumoxid, Calciumoxid. Außerdem können organische Basen wie Pyridin oder tertiäre Amine Verwendung finden. Die Base wird beispielsweise in einer Menge von 0,5 bis 2 Mol-Äquivalent, bezogen auf die Ammoniumverbindung, zugesetzt.

Als Lösungsmittel eignen sich beispielsweise Dimethylsulfoxid; Alkohole wie Methanol, Ethanol und Isopropanol; aromatische Kohlenwasserstoffe wie Benzol und Toluol; chlorierte Kohlenwasserstoffe wie Chloroform und Dichlorethan; aliphatische Kohlenwasserstoffe wie Hexan und Cyclohexan; Ester wie Essigsäureethylester und Ether wie Diethylether, Dioxan und Tetrahydrofuran. Vorzugsweise führt man die Umsetzung in Methanol mit Natriumhydrogencarbonat als Base durch.

Die Reaktion ist nach wenigen Stunden beendet. Die Zielverbindung kann z.B. durch Einengen der Mischung, Verteilung des Rückstandes in Methylenchlorid/Wasser und Abdestillieren des Lösungsmittels unter vermindertem Druck isoliert werden.

Man kann für diese Umsetzung aber auch unmittelbar die freie Hydroxylaminbase, z.B. in Form einer wäßrigen Lösung, verwenden; je nach verwendetem Lösungsmittel für die Verbindung II erhält man ein ein- oder zweiphasiges Reaktionsgemisch.

Geeignete Lösungsmittel für diese Variante sind beispielsweise Alkohole wie Methanol, Ethanol, Isopropanol und Cyclohexanol; aliphatische und aromatische, gegebenenfalls chlorierte Kohlenwasserstoffe wie Hexan, Cyclohexan, Methylenchlorid, Toluol und Dichlorethan; Ester wie Essigsäureethylester; Nitrile wie Acetonitril und cyclische Ether wie Dioxan und Tetrahydrofuran.

Alkalimetallsalze der Verbindungen I können durch Behandeln der 3-Hydroxy-verbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer-, Zink-, Eisen-, Calcium-, Magnesium- und Bariumsalze können aus den Natriumsalzen in üblicher Weise hergestellt werden, ebenso Ammonium- und Phosphoniumsalze mittels Ammoniak, Phosphonium-, Sulfonium- oder Sulfoxoniumhydroxiden.

Die Verbindungen des Typs II können z.B. aus den entsprechenden Cyclohexan-1,3-dionen der Formel IV 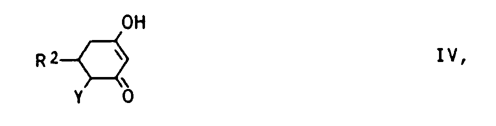
in der
- Y: Wasserstoff oder Methoxycarbonyl bedeutet
nach bekannten Methoden, z.B. wie in Tetrahedron Lett., 2491 (1975) beschrieben, hergestellt werden.

Es ist auch möglich, die Verbindungen der Formel II über die Zwischenstufe der Enolester V herzustellen, die bei der Umsetzung von Verbindungen der Formel IV mit Säurechloriden VI in Gegenwart von Basen anfallen und anschließend mit bestimmten Imidazol- oder Pyridinderivaten umgelagert werden (JP-OS 79/063 052).

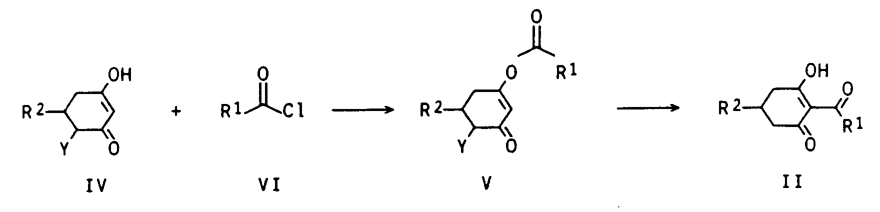

Zu den Verbindungen der Formel IV gelangt man über eine Reihe bekannter Verfahrensschritte ausgehend von bekannten Vorprodukten.

Die Synthese der Hydroxylamine III erfolgt gemäß dem nachstehenden Reaktionsschema beispielsweise über
A) die Alkylierung von N-Hydroxyphthalimid VII mit geeigneten Phenylbutylhalogeniden VIII und anschließende Schutzgruppenabspaltung beispielsweise mit Hydrazin oder Ethanolamin analog Beispielen aus EP-A-244 786 bzw. Houben-Weyl, Methoden der organischen Chemie, Band X/1, Seite 1152ff.
B) Hydrierung von N-4-Phenylbutenyloxyphthalimiden Xa,b, deren Herstellung in DE-A 38 38 310 beschrieben ist, mittels geeigneten Katalysatoren wie z.B. Palladium auf Aktivkohle, in geeigneten inerten Lösungsmitteln wie z.B. Methanol, Tetrahydrofuran, Dioxan und anschließende Schutzgruppenabspaltung wie voranstehend beschrieben.
   Vorteilhaft wird die Hydrierung bei Temperaturen von 20°C bis zum Siedepunkt des Lösungsmittels, insbesondere bei Raumtemperatur nach den dafür üblichen Techniken Atmosphärendruck, über- oder Unterdruck durchgeführt. Bevorzugt ist ein Druckbereich von 1 bis 10, insbesondere 1 bis 2 bar.

### Reaktionsschema:

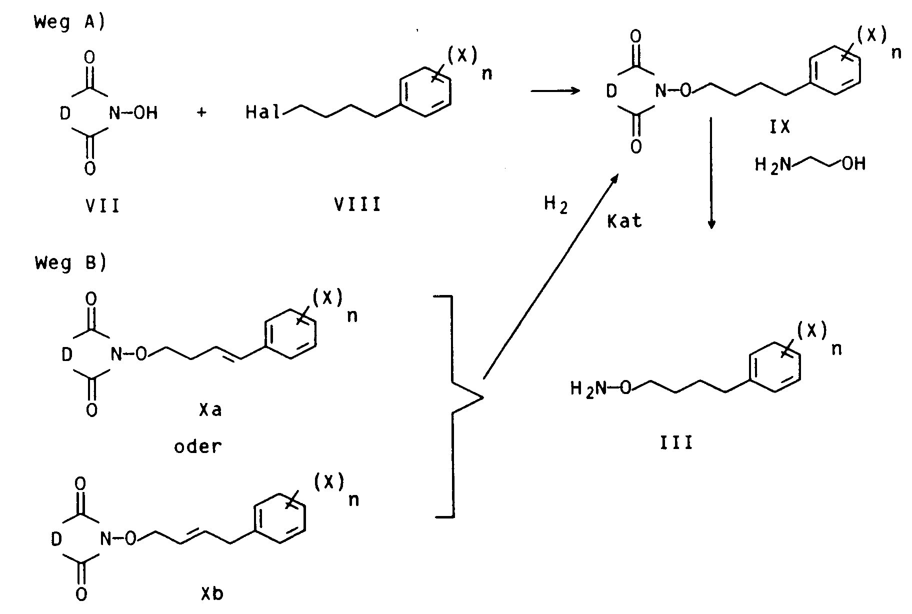

In den cyclischen Hydroxyimiden VII steht D z.B. für C₂-C₃-Alkylen, C₂-Alkenylen oder einen mit bis zu 3 Doppelbindungen und gegebenenfalls 1 Stickstoffatom enthaltenden 5- oder 6-Ring, z.B. für Phenylen, Pyridinylen, Cyclopentylen, Cyclohexylen oder Cyclohexenylen. Beispielsweise kommen folgende Substanzen in Betracht:

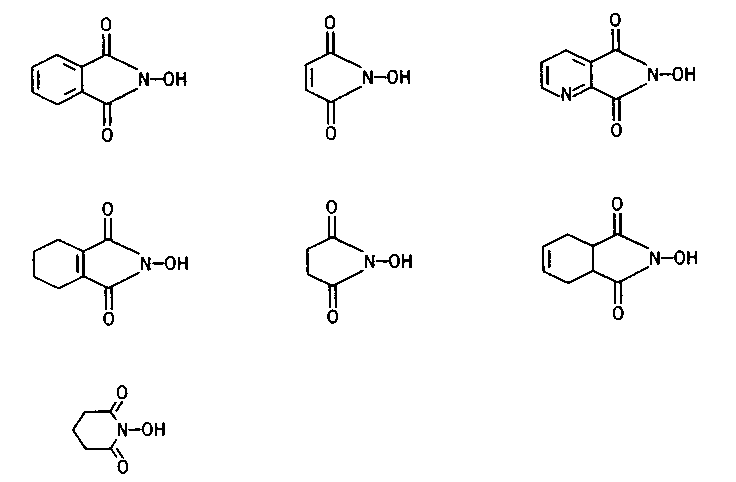

Die Spaltung der cyclischen Imidether VIII gelingt analog einem in EP-A 244 786 beschriebenen Verfahren mit Alkanolaminen. Die Hydroxylamine III können nach diesem Verfahren als freie Basen oder nach Fällung mit Säuren als Salze isoliert werden. Gut kristallisierende Salze erhält man durch Umsetzung der Basen mit Oxalsäure.

Im Hinblick auf die biologische Wirksamkeit werden Cyclohexenone der Formel I bevorzugt, in denen die Substituenten folgende Bedeutung haben:
- R¹: Alkyl wie Methyl, Ethyl, Propyl, n-Butyl, insbesondere Ethyl und Propyl;
- X: Halogen wie Fluor, Chlor, Brom und Iod, insbesondere Fluor und Chlor;
- n: 1 bis 5, insbesondere 1, 2 oder 3. Bei mehreren Resten X können die Substituenten gleich oder verschieden sein.
- R²: Alkyl wie unter R¹ genannt, welches eine unten aufgeführten Alkoxy- oder Alkylthiogruppen bevorzugt in 1-, 2- oder 3-Position tragen kann, insbesondere 2-Ethylthiopropyl, oder einen Cyclohexylrest, der 1 bis 3 Methyl- oder Hydroxygruppen tragen kann, insbesondere 4-Methylcyclohexyl und 3,4-Dihydroxycyclohexyl;
5-gliedriges Heteroaryl wie Pyrazolyl, Isoxazolyl;
ein 6-gliedriger Heterocyclus wie Tetrahydropyran-3-yl, Tetrahydropyran-4-yl und Tetrahydrothiopyran-3-yl,
ein Phenyl- oder ein Pyridylrest,
wobei die cyclischen Reste ein bis drei Alkylgruppen, Alkoxygruppen, Alkylthiogruppen und/oder Halogenalkylgruppen sowie im Fall 6-gliedriger Reste auch Halogen wie unter X genannt oder Hydroxy tragen können, beispielsweise
Alkyl wie Methyl, Ethyl, Propyl, 1-Methylethyl, Butyl, 1-Methylpropyl, 2-Methylpropyl und 1,1-Dimethylethyl, insbesondere Methyl und 1,1-Dimethylethyl,
Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy,
Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio,
Halogenalkyl wie Fluormethyl, Difluormethyl, Trifluormethyl, Chlordifluormethyl, Dichlorfluormethyl, Trichlormethyl, insbesondere Difluormethyl, Trifuormethyl.
Besonders bevorzugt ist die 2,4,6-Trimethylphenylgruppe.
Die 5-gliedrigen Heteroaromaten in der Bedeutung R² können als Substituenten folgende Reste tragen:
Halogenatom wie unter X genannt, insbesondere Fluor und Chlor,
Bei den Phenyl- und Pyridylresten kommen als Substituenten neben den obengenannten Gruppen auch folgende Reste in Betracht:
Alkinyloxy wie 2-Propinyloxy, insbesondere Propargyloxyphenyl.
Amino, welches ein oder zwei Acylreste wie Acetyl oder Benzoyl tragen kann.
Besonders bevorzugte Cyclohexenonoximether der Formel I sind in folgender Tabelle zusammengefaßt:

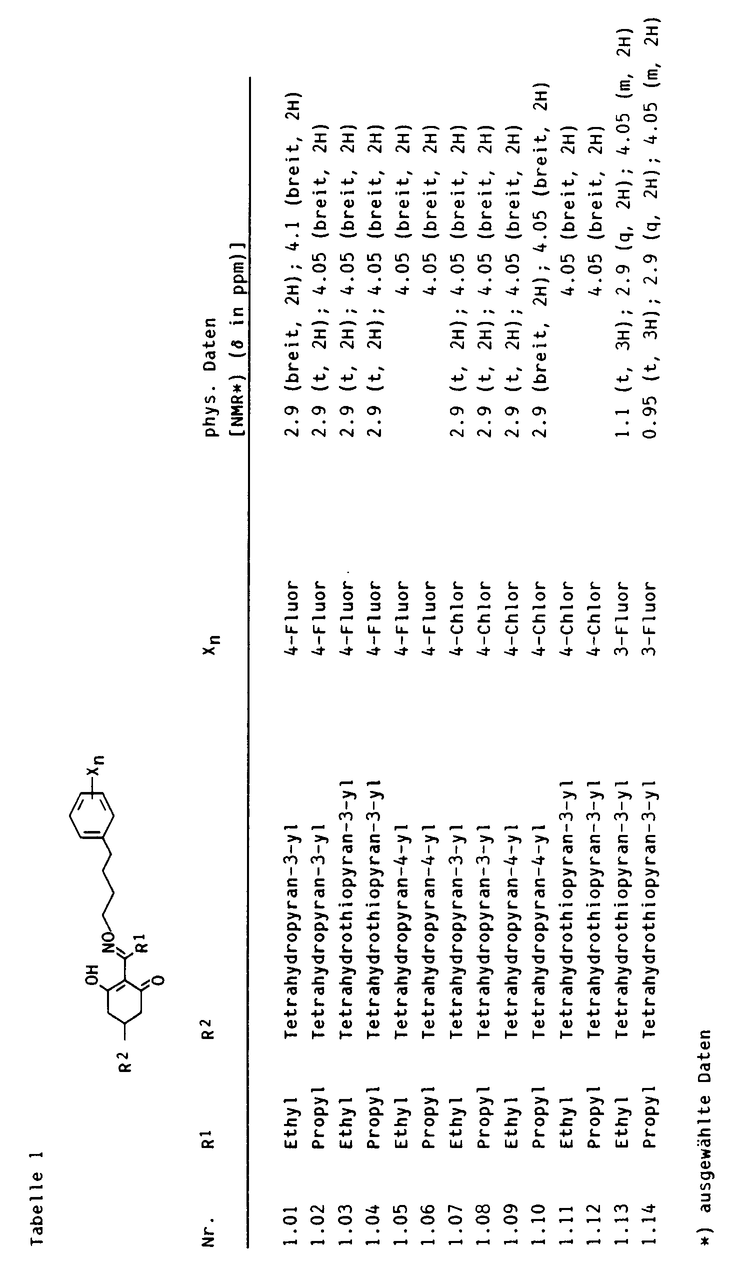

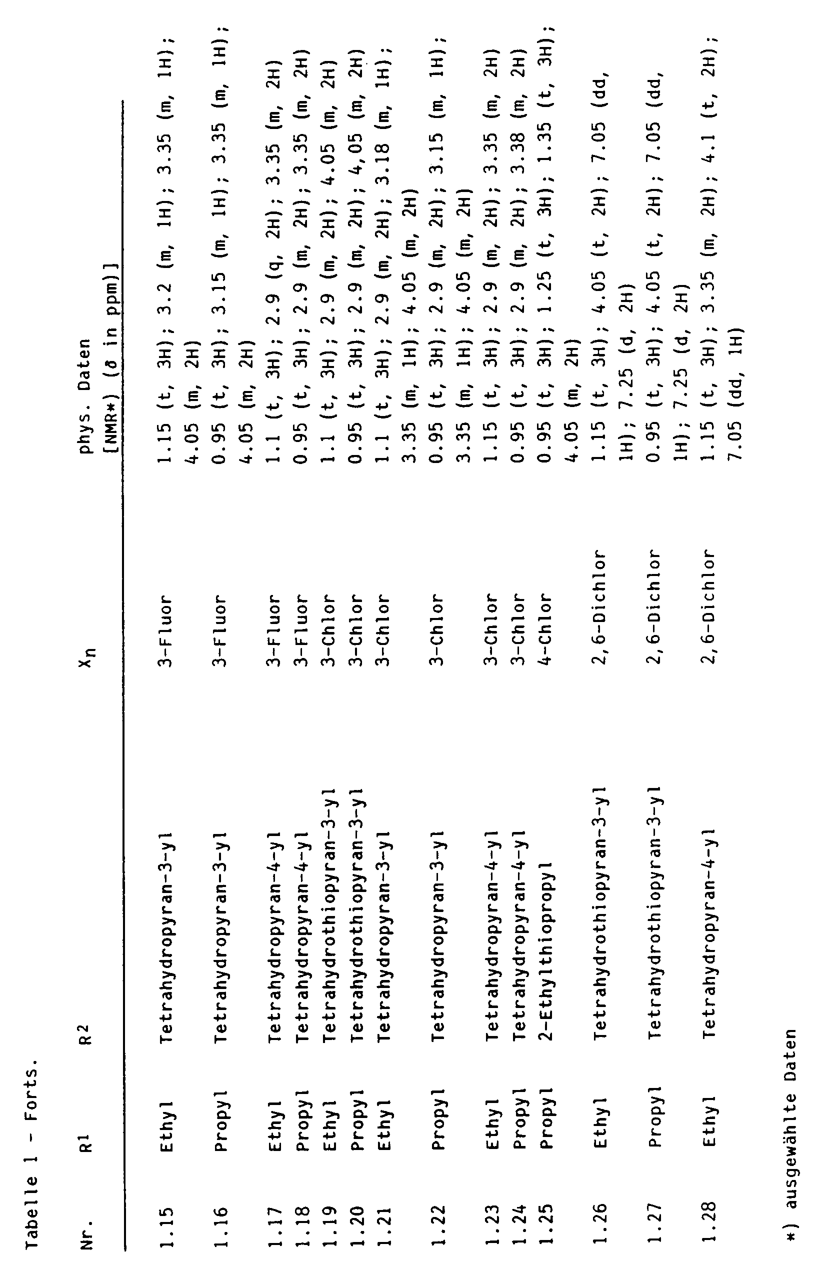

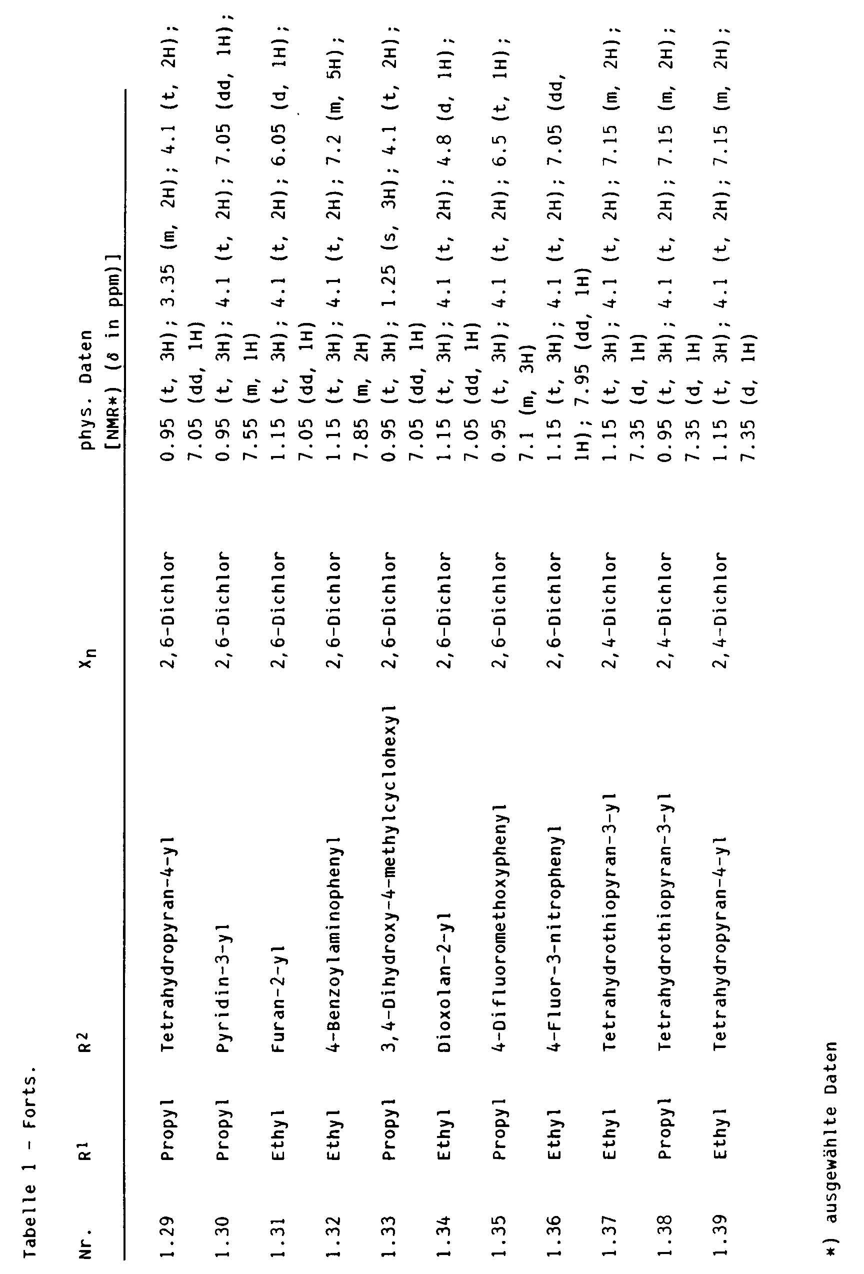

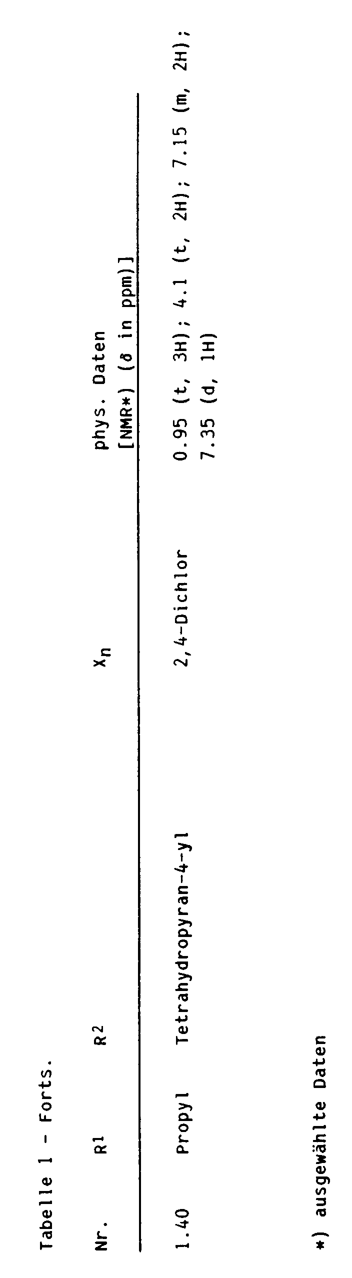

Die Cyclohexenonoximether I eignen sich als Herbizide, insbesondere zur Bekämpfung von Pflanzenarten aus der Familie der Gramineen (Gräser).

Die Cyclohexenonoximether I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,02 und 95 Gew.% vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I. Man vermischt 90 Gewichtsteile der Verbindung Nr. 1.05 mit 10 Gewichtsteilen N-Methyl-α-pyrrolidon und erhält eine Lösung, die zur Anwendung in Form kleinster Tropfen geeignet ist.
II. 20 Gewichtsteile der Verbindung Nr. 1.03 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
III. 20 Gewichtsteile der Verbindung Nr. 1.11 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. 1.11 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
V. 20 Gewichtsteile des Wirkstoffs Nr. 1.05 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.
VI. 3 Gewichtsteile des Wirkstoffs Nr. 1.03 werden mit 97 Gewichtsteilen feinteiligem Kaolin vermischt. Man erhält auf diese Weise ein Stäubemittel, das 3 Gew.% des Wirkstoffs enthält.
VII. 30 Gewichtsteile des Wirkstoffs Nr. 1.05 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 20 Gewichtsteile des Wirkstoffs Nr. 1.03 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der Mittel kann im Vorauflauf- oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,001 bis 3 kg/ha, vorzugsweise 0,01 bis 2,0 kg/ha.

In Anbetracht des erfaßbaren Wirkungsspektrums zur Unkrautbekämpfung, der Verträglichkeit für Kulturpflanzen oder der erwünschten Beeinflussung des Wachstums derselben sowie angesichts der Vielfalt der Applikationsmethoden können die erfindungsgemäßen Verbindungen in einer großen Zahl von Kulturpflanzen eingesetzt werden. In Betracht kommen beispielsweise folgende Kulturen:

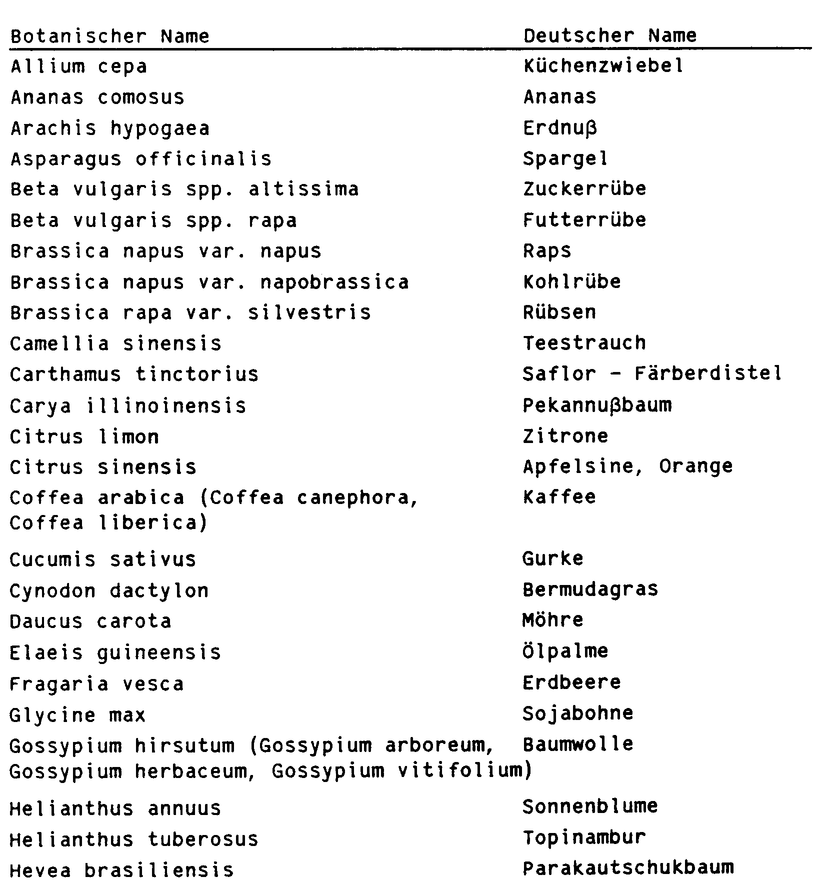

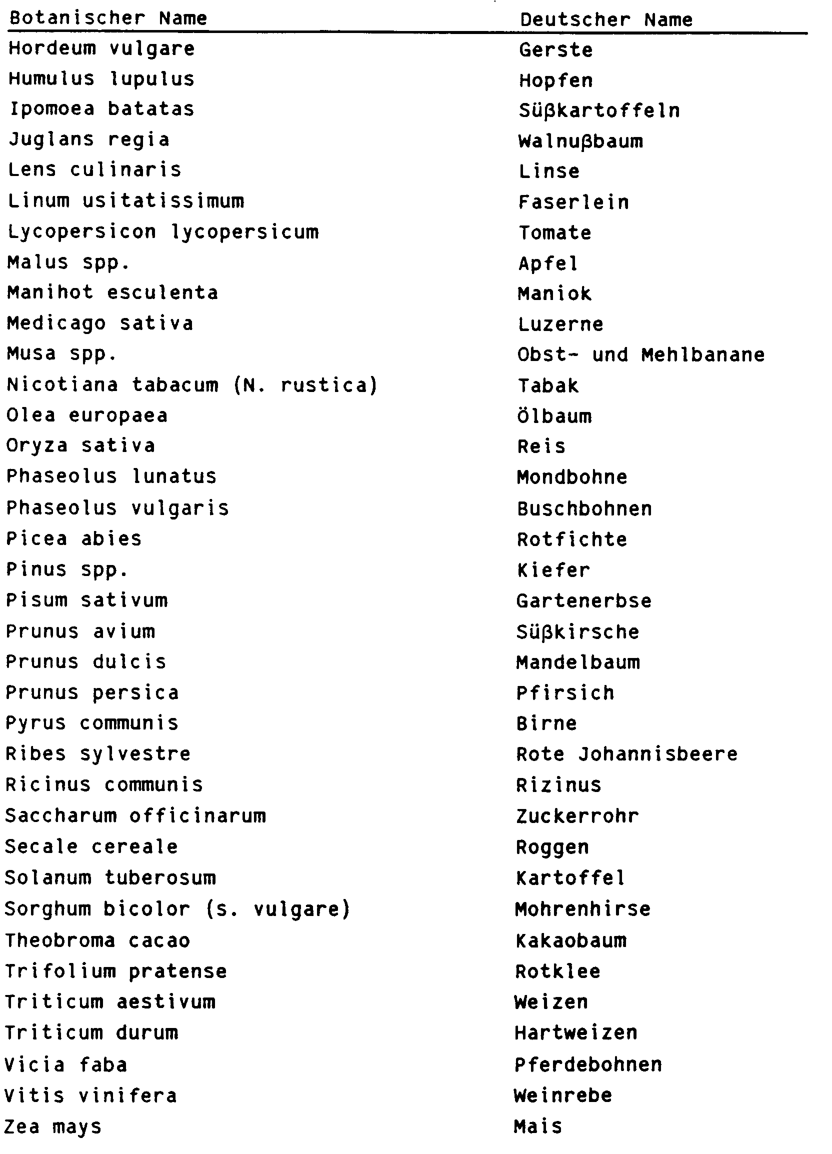

Zur Verbreiterung des Wirkungsspektrums und zur Erzielung synergistischer Effekte können die Cyclohexenonderivate der Formel I sowohl untereinander als auch mit Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren, Sulfonylharnstoffderivate, Cyclohexenone, (Hetero)aryloxyphenoxypropionsäuren, deren Salze, Ester und Amide und andere in Betracht.

Außerdem kann es von Nutzen sein, die Cyclohexenonderivate der Formel I bzw. sie enthaltende herbide Mittel allein oder in Kombination mit anderen Herbiziden oder auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Beispiele

4-Phenylbutyloxyamin

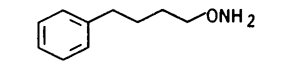

### Methode A:

Zu 302 g (1,85 mol) N-Hydroxyphthalimid in 1,9 l wasserfreiem N-Methylpyrrolidon wurden 167,5 g (1,21 mol) Kaliumcarbonat und 2 g Kaliumjodid gegeben. Nachdem man die Reaktionsmischung auf 60°C erwärmt hatte, tropfte man 395,2 g (1,85 mol) 4-Phenylbutylbromid zu und hielt noch 6 h bei dieser Temperatur. Nach Abkühlen goß man auf 6 l Eiswasser, nahm mit Dichlorethan auf, wusch die organische Phase mit verdünnter Natronlauge, trocknete und engte im Vakuum ein. Ausbeute: 397 g N-(4-Phenylbutyloxy)-phthalimid (73 %).
250-MHz ¹H-NMR (DMSO-d₆)
δ(ppm) = 1,65-1,85 (m, 4H); 2,66 (t, 2H); 4,18 (t, 2H)
7,1-7,4 (m, 5H); 7,86 (s, 4H).

Zu eienr Lösung aus 396 g (1,34 mol) des oben erhaltenen Phthalimidethers in 1300 ml Essigester wurden 81,8 g (1,34 mol) Ethanolamin gegeben. Man erhitzte die Reaktionsmischung 5 h lang auf 60°C. Danach rührte man noch 24 h bei Raumtemperatur, saugte die ausgefallenen Kristalle ab, wusch mit wenig Essigester nach und leitete durch die vereinten Mutterlaugen 15 min lang einen kräftigen Salzsäuregassstrom, wobei die Innentemperatur 40°C nicht übersteigen sollte. Man saugte die ausgefallenen Kristalle ab, wusch mit wenig Essigester nach und trocknete im Vakuum. Ausbeute: 148,5 g 4-Phenylbutyloxyamin-Hydrochlorid (55 %); Fp.: 93-94°C.
250-MHz ¹H-NMR (DMSO-d₆)
α(ppm) = 1,5-1,7 (m, 4H); 2,58 (t, 2H); 4,04 (t, 2H)
7,1-7,3 (m, 5H); 11,1 (breites s, 3H).

### 4-(4-Fluorphenyl)butyloxyamin

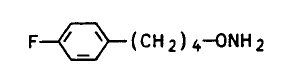

### Methode B: Hydrierung

### N-(4-(4-Fluorphenyl)butyloxy)phthalimid

Zu einer Lösung von 71,5 g (0,23 mol) N-(4-(4-Fluorphenyl)-3-butenyloxy)-phthalimid (hergestellt nach DE-OS 38 38 310) in 300 ml Tetrahydrofuran wurden 2 g Palladium auf Aktivkohle (10 %ig) gegeben. Man hydrierte bei leichtem Überdruck bis das 1,2-fache der theoretischen Wasserstoffmenge verbraucht worden war. Man saugte über Kieselgur ab, engte ein und kristallisierte das Rohprodukt aus Isopropanol um.
Ausbeute: 62,8 g (87 %); Fp.: 67-68°C
250-MHz ¹H-NMR (DMSO-d₆)
δ(ppm) = 1,8-1,9 (m, 4H); 2,65 (t, 2H); 4,15 (t, 2H)
7,0-7,35 (m, 4H); 7,86 (s, 4H).

61,8 g (0,197 mol) des zuvor hergestellten Phthalimidethers wurden portionsweise in 92 mol Ethanolamin eingetragen. Nachdem man 3 h auf 60°C erhitzt hatte, goß man nach Erkalten in 400 ml Eiswasser und extrahierte mit Dichlorethan. Die vereinten organischen Phasen wurden mit gesättigter Kochsalzlösung gewaschen, getrocknet und im Vakuum eingeengt. Man erhielt das 4-(4-Fluorphenyl)butyloxyamin als Öl.
250-MHz ¹H-NMR (CDCl₃)
δ(ppm) = 1,5-1,75 (m, 4H); 2,61 (t, 2H); 3,68 (t, 2H)
5,4 (breites s, 2H); 6,9-7,2 (m, 4H).

### 4-(4-Chlorphenyl)butyloxyamin

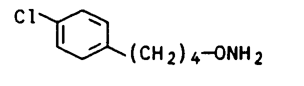

Analog dem zuvor beschriebenen Beispiel erhielt man ausgehend von N-(4-(4-Chlorphenyl)-2-butenyloxyphthalimid (DE-OS 38 38 310) in einer Gesamtausbeute von 60 % das 4-(4-Chlorphenyl)butyloxyamin als Öl.
250-MHz ¹H-NMR (CDCl₃)
δ(ppm) = 1,65-1,85 (m, 4H); 2,66 (t, 2H); 4,18 (t, 2H)
7,1-7,4 (m, 5H); M 7,86 (s, 4H).

### N-(4-(4-Chlorphenyl)-butyloxyphthalimid

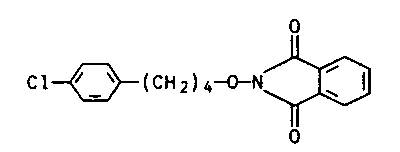

Fp.: 72-73°C Isopropanol
250-MHz ¹H-NMR (DMSO-d₆)
δ(ppm) = 1,6-1,85 (m, 4H); 2,68 (t, 2H); 4,17 (t, 2H)
7,2-7,4 (m, 4H); 7,88 (s, 4H).

### 2-{1-[4-(4-Fluorphenyl)-butyloximino]-butyl}-5-tetrahydrothiopyran-3-yl-3-hydroxy-cyclohex-2-enon (Verbindung 1.04)

Eine Lösung aus 3 g (11 mol) 2-Butyryl-3-hydroxy-5-tetrahydrothiopyran-3-yl-cyclohex-2-enon in 100 ml trockenem Methanol wurde mit 2,2 g (12 mmol) 4-(4-Fluorphenyl)-butoxyamin versetzt. Das Gemisch rührte 16 h bei Raumtemperatur und wurde dann im Vakuum zur Trockene eingedampft. Der Rückstand wurde in Diethylether aufgenommen und an Kieselgel chromatographiert. Ausbeute: 3,6 g (80 % d.Th.).

Entsprechend dieser Vorschrift können die in Tabelle 1 aufgeführten Cyclohexenonverbindungen erhalten werden.

### Anwendungsbeispiele

Die herbizide Wirkung der Cyclohexenoximether der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,06 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Latenischer Name | Deutscher Name | Englischer Name |
|---|---|---|
| Orvza sativa | Reis | rice |
| Echinochloa crus-galli | Hühnerhirse | barnyardgrass |
| Setaria italica | Kolbenhirse | foxtail millet |

Mit 0,06 kg/ha a.S. im Nachauflaufverfahren eingesetzt, lassen sich mit den Beispielen 1.11, 1.03 und 1.05 unerwünschte grasartige Pflanzen sehr gut bekämpfen, bei gleichzeitiger Verträglichkeit an der Beispielkultur Reis.

## Patentansprüche (Patentansprüche für folgende(n) Vertragsstaat(en): BE, CH, DE, FR, GB, IT, LI, NL)

1. Cyclohexenonoximether der allgemeinen Formel I 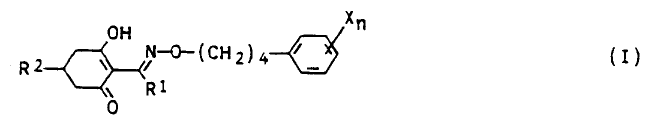 in der die Variablen folgende Bedeutung haben:
R¹ eine C₁-C₆-Alkylgruppe;
X Halogen;
n 1 bis 5;
R² Tetrahydropyran-3-yl, Tetrahydropyran-4-yl, Tetrahydrothiopyran-3-yl
R³ Wasserstoff, eine C₁-C₄-Alkylgruppe, eine C₃-C₆-Alkenylgruppe oder eine C₃-C₆-Alkinylgruppe und
R⁴ Wasserstoff, eine C₁-C₄-Alkylgruppe, eine C₃-C₆-Alkenylgruppe, eine C₃-C₆-Alkinylgruppe, eine C₁-C₆-Acylgruppe oder ein Benzoylrest, wobei der aromatische Ring noch einen bis drei Reste tragen kann, ausgewählt aus einer Gruppe, bestehend aus Nitro, Cyano, Halogen, C₁-C₄-Alkyl, C₁-C₄-Alkoxy, C₁-C₄-Alkylthio und C₁-C₄-Halogenalkyl,
bedeuten,
sowie die landwirtschaftlich nutzbaren Salze von I und die Ester von I mit C₁-C₁₀-Carbonsäuren oder anorganischen Säuren,

2. Herbizides Mittel, enthaltend eine herbizid wirksame Menge mindestens einer Verbindung der Formel I gemäß Anspruch 1 und inerte Zusatzstoffe.

3. Verfahren zur Bekämpfung von unerwünschtem Pflanzenwuchs, dadurch gekennzeichnet, daß man die unerwünschten Pflanzen und/oder ihren Lebensraum miteiner herbizid wirksamen Menge eines Cyclohexenonoximethers der Formel I gemäß Anspruch 1 behandelt.

## Patentansprüche (Patentansprüche für folgende(n) Vertragsstaat(en): AT)

1. Cyclohexenonoximether der allgemeinen Formel I 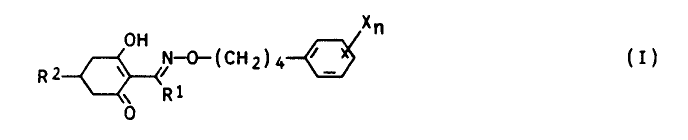 in der die Variablen folgende Bedeutung haben:
R¹ eine C₁-C₆-Alkylgruppe;
X Halogen;
n 1 bis 5;
R² eine C₁-C₄-Alkoxy-C₁-C₆-alkyl- oder C₁-C₄-Alkylthio-C₁-C₆-alkylgruppe
eine C₃-C₇-Cycloalkylgruppe oder eine C₅-C₇-Cycloalkenylgruppe, wobei diese Gruppen noch einen bis drei Reste tragen können, ausgewählt aus einer Gruppe, bestehend aus C₁-C₄-Alkyl, C₁-C₄-Alkoxy, C₁-C₄-Alkylthio, C₁-C₄-Halogenalkyl, Hydroxy und Halogen;
ein 5-gliedriger gesättigter Heterocyclus der ein oder zwei Heteroatome, ausgewählt aus einer Gruppe bestehend aus Sauerstoff und Schwefel, enthält, und der noch einen bis drei Reste tragen kann, ausgewählt aus einer Gruppe, bestehend aus C₁-C₄-Alkyl, C₁-C₄-Alkoxy, C₁-C₄-Alkylthio und C₁-C₄-Halogenalkyl;
ein 6- oder 7-gliedriger gesättigter, ein- oder zweifach ungesättigter Heterocyclus, enthaltend ein oder zwei Heteroatome, ausgewählt aus einer Gruppe bestehend aus Sauerstoff und Schwefel, wobei der Heterocyclus noch einen bis drei Reste tragen kann, ausgewählt aus einer Gruppe, bestehend aus Hydroxy, Halogen, C₁-C₄-Alkyl, C₁-C₄-Alkoxy, C₁-C₄-Alkylthio und C₁-C₄-Halogenalkyl;
ein 5-gliedriger Heteroaromat, enthaltend ein bis drei Heteroatome, ausgewählt aus einer Gruppe bestehend aus zwei Stickstoffatomen und einem Sauerstoff- oder Schwefelatom, wobei dieser Ring noch einen bis drei Reste tragen kann, ausgewählt aus einer Gruppe, bestehend aus Halogen, Cyano, C₁-C₄-Alkyl, C₁-C₄-Alkoxy, C₁-C₄-Alkylthio, C₁-C₄-Halogenalkyl, C₂-C₆-Alkenyl, C₂-C₆-Alkenyloxy, C₂-C₆-Halogenalkenyl und C₁-C₄-Alkoxy-C₁-C₄-alkyl;
die Phenylgruppe oder die Pyridylgruppe, wobei diese Gruppen noch einen bis drei Reste tragen können, ausgewählt aus einer Gruppe, bestehend aus Halogen, Nitro, Cyano, C₁-C₄-Alkyl, C₁-C₄-Alkoxy, C₁-C₄-Alkylthio, C₁-C₄-Halogenalkyl, C₃-C₆-Alkenyloxy, C₃-C₆-Alkinyloxy und -NR³R⁴, worin
R³ Wasserstoff, eine C₁-C₄-Alkylgruppe, eine C₃-C₆-Alkenylgruppe oder eine C₃-C₆-Alkinylgruppe und
R⁴ Wasserstoff, eine C₁-C₄-Alkylgruppe, eine C₃-C₆-Alkenylgruppe, eine C₃-C₆-Alkinylgruppe, eine C₁-C₆-Acylgruppe oder ein Benzoylrest, wobei der aromatische Ring noch einen bis drei Reste tragen kann, ausgewählt aus einer Gruppe, bestehend aus Nitro, Cyano, Halogen, C₁-C₄-Alkyl, C₁-C₄-Alkoxy, C₁-C₄-Alkylthio und C₁-C₄-Halogenalkyl,
bedeuten,
sowie ihre landwirtschaftlich nutzbaren Salze und Ester von C₁-C₁₀-Carbonsäuren und anorganischen Säuren.

2. Herbizides Mittel, enthaltend eine herbizid wirksame Menge mindestens einer Verbindung der Formel I gemäß Anspruch 1 und inerte Zusatzstoffe.

3. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Cyclohexenonderivates der Formel I gemäß Anspruch 1 behandelt.

## Patentansprüche (Patentansprüche für folgende(n) Vertragsstaat(en): ES)

1. Verfahren zur Herstellung von Cyclohexenonoximethen der allgemeinen Formel I 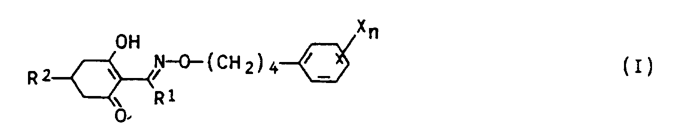 in der die Variablen folgende Bedeutung haben:
R¹ eine C₁-C₆-Alkylgruppe;
X Halogen;
n 1 bis 5:
R² Tetrahydropyran-3-yl, Tetrahydropyran-4-yl, Tetrahydrothiopyran-3-yl
R³ Wasserstoff, eine C₁-C₄-Alkylgruppe, eine C₃-C₆-Alkenylgruppe oder eine C₃-C₆-Alkinylgruppe und
R⁴ Wasserstoff, eine C₁-C₄-Alkylgruppe, eine C₃-C₆-Alkenylgruppe, eine C₃-C₆-Alkinylgruppe, eine C₁-C₆-Acylgruppe oder ein Benzoylrest, wobei der aromatische Ring noch einen bis drei Reste tragen kann, ausgewählt aus einer Gruppe, bestehend aus Nitro, Cyano, Halogen, C₁-C₄-Alkyl, C₁-C₄-Alkoxy, C₁-C₄-Alkylthio und C₁-C₄-Halogenalkyl,
bedeuten,
sowie die landwirtschaftlich nutzbaren Salze von I und die Ester von I mit C₁-C₁₀-Carbonsäuren oder anorganischen Säuren,
dadurch gekennzeichnet, daß man ein Cyclohexenon der Formel II 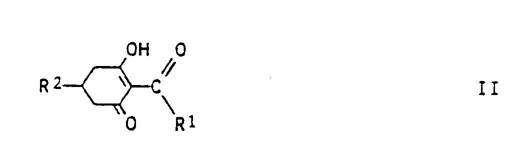 mit einem Hydroxylamin der Formel III 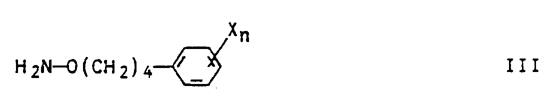 oder mit einem seiner Salze, gewünschtenfalls in Gegenwart einer Base, umsetzt,
und die Verfahrensprodukte I gewünschtenfalls in ihre landwirtschaftlich nutzbaren Salze oder Ester von C₁-C₁₀-Carbonsäuren oder organischen Säuren überführt.

2. Herbizides Mittel, enthaltend eine herbizid wirksame Menge mindestens einer Verbindung der Formel I gemäß Anspruch I und inerte Zusatzstoffe.

3. Verfahren zur Bekämpfung von unerwünschtem Pflanzenwuchs, dadurch gekennzeichnet, daß man die unerwünschten Pflanzen und/oder ihren Lebensraum mit einer herbizid wirksamen Menge eines Cyclohexenonoximethers der Formel I gemäß Anspruch 1 behandelt.

## Claims (Claims for the following Contracting State(s): BE, CH, DE, FR, GB, IT, LI, NL)

1. A cyclohexenone oxime ether of the formula I 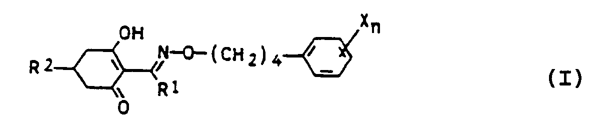 where R¹ is C₁-C₆-alkyl,
X is halogen,
n is from 1 to 5 and
R² is tetrahydropyran-3-yl, tetrahydropyran-4-yl or tetrahydrothiopyran-3-yl,
and the agriculturally usable salts of I and the esters of I with C₁-C₁₀-carboxylic acids or inorganic acids.

2. A herbicide containing a herbicidal amount of one or more compounds of the formula I as claimed in claim 1 and inert additives.

3. A method for controlling undesirable plant growth, wherein the undesirable plants and/or their habitat are treated with a herbicidal amount of a cyclohexenone oxime ether of the formula I as claimed in claim 1.

## Claims (Claims for the following Contracting State(s): AT)

1. A cyclohexenone oxime ether of the formula I 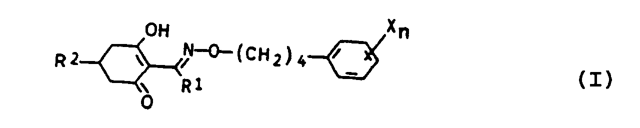 where R¹ is C₁-C₆-alkyl,
X is halogen,
n is from 1 to 5,
R² is C₁-C₄-alkoxy-C₁-C₆-alkyl or C₁-C₄-alkylthio-C₁-C₆-alkyl,
C₃-C₇-cycloalkyl or C₅-C₇-cycloalkenyl, and these groups may additionally carry from one to three radicals selected from the group consisting of C₁-C₄-alkyl, C₁-C₄-alkoxy, C₁-C₄-alkylthio, C₁-C₄-haloalkyl, hydroxyl and halogen,
a 5-membered saturated heterocyclic structure which contains one or two hetero atoms chosen from oxygen or sulfur and may additionally carry from one to three radicals selected from the group consisting of C₁-C₄-alkyl, C₁-C₄-alkoxy, C₁-C₄-alkylthio and C₁-C₄-haloalkyl,
a 6-membered or 7-membered saturated or mono- or diunsaturated heterocyclic structure containing one or two hetero atoms selected from the group consisting of oxygen and sulfur, and the heterocyclic structure may additionally carry from one to three radicals selected from the group consisting of hydroxyl, halogen, C₁-C₄-alkyl, C₁-C₄-alkoxy, C₁-C₄-alkylthio and C₁-C₄-haloalkyl, a 5-membered heteroaromatic structure containing one to three hetero atoms selected from the group consisting of two nitrogen atoms and one oxygenor sulfur atom, and this ring may additionally carry from one to three radicals selected from the group consisting of halogen, cyano, C₁-C₄-alkyl, C₁-C₄-alkoxy, C₁-C₄-alkylthio, C₁-C₄-haloalkyl, C₂-C₆-alkenyl, C₂-C₆-alkenyloxy, C₂-C₆-haloalkenyl and C₁-C₄-alkoxy-C₁-C₄-alkyl,
phenyl or pyridyl, and these groups may additionally carry from one to three radicals selected from the group consisting of halogen, nitro, cyano, C₁-C₄-alkyl, C₁-C₄-alkoxy, C₁-C₄-alkylthio, C₁-C₄-haloalkyl, C₃-C₆-alkenyloxy, C₃-C₆-alkynyloxy and -NR³R⁴, where
R³ is hydrogen, C₁-C₄-alkyl, C₃-C₆-alkenyl or C₃-C₆-alkynyl and
R⁴ is hydrogen, C₁-C₄-alkyl, C₃-C₆-alkenyl, C₃-C₆-alkynyl, C₁-C₆-acyl or benzoyl, and the aromaticring may additionally carry from one to three radicals selected from the group consisting of nitro, cyano, halogen, C₁-C₄ alkyl, C₁-C₄-alkoxy, C₁-C₄-alkylthio and C₁-C₄-haloalkyl, and its agriculturally usable salts and esters of C₁-C₁₀-carboxylic acids andin organic acids.

2. A herbicide containing a herbicidal amount of one or more compounds of the formula I as claimed in claim 1 and inert additives.

3. A method for controlling undesirable plant growth, wherein the undesirable plants and/or their habitat are treated with a herbicidal amount of a cyclohexenone derivative of the formula I as claimed in claim 1.

## Claims (Claims for the following Contracting State(s): ES)

1. A process for the preparation of a cyclohexenone oxime ether of the formula I 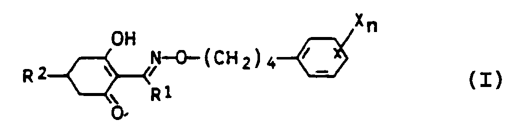 where R¹ is C₁-C₆-alkyl,
X is halogen,
n is from 1 to 5 and
R² is tetrahydropyran-3-yl, tetrahydropyran-4-yl or tetrahydrothiopyran-3-yl,
and the agriculturally usable salts of I and the esters of I with C₁-C₁₀-carboxylic acids or inorganic acids, wherein a cyclohexenone of the formula II 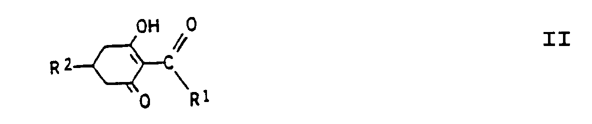 is reacted with a hydroxylamine of the formula III 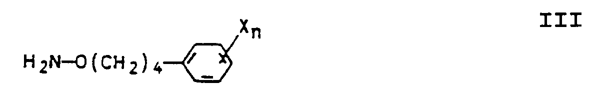 or with one of its salts, if desired in the presence of a base, and the products I are, if desired, converted into their agriculturally usable salts or esters of C₁-C₁₀-carboxylic acids or inorganic acids.

2. A herbicide containing a herbicidal amount of one or more compounds of the formula I as claimed in claim 1 and inert additives.

3. A method for controlling undesirable plant growth, wherein the undesirable plants and/or their habitat are treated with a herbicidal amount of a cyclohexenone oxime ether of the formula I as claimed in claim 1.

## Revendications (Revendications pour l'(les) Etat(s) contractant(s) suivant(s): BE, CH, DE, FR, GB, IT, LI, NL)

1. Cyclohexénonoximéther de formule générale I 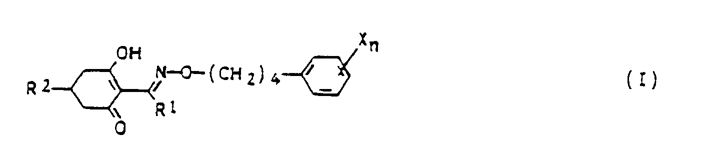 dans laquelle les variables ont les significations suivantes :
H¹ représente un groupe alkyle en C₁-C₆ ;
X représente un atome d'halogène ;
n présente une valeur de 1 à 5 ;
R² représente un groupe tétrahydropyran-3-yle, tétrahydropyran-4-yle, tétrahydrothiopyran-3-yle,
ainsi que les sels et esters du composé de formule I, formés avec des acides carboxyliques en C₁-C₁₀ et des acides inorganiques, utilisables en agriculture.

2. Agent herbicide, contenant une quantité ayant une activité herbicide d'au moins un composé de formule I selon la revendication 1, et des adjuvants inertes.

3. Procédé de lutte contre des végétations indésirables, caractérisé par le fait que les végétations indésirables et/ou leur espace vital sont traités avec une quantité ayant une activité herbicide d'un cyclohexénonoximéther de formule I selon la revendication 1.

## Revendications (Revendications pour l'(les) Etat(s) contractant(s) suivant(s): AT)

1. Cyclohexénonoximéther de formule générale I 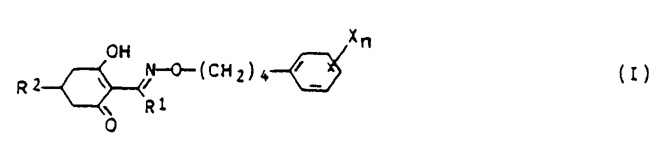 dans laquelle les variables ont les significations suivantes :
R¹ représente un groupe alkyle en C₁-C₆ ;
X représente un atome d'halogène ;
n présente une valeur de 1 à 5 ;
R² représente un groupe alcoxy(C₁-C₄)-alkyle(C₁-C₆) ou alkylthio(C₁-C₄)-alkyle(C₁-C₆) ;
un groupe cycloaikyle en C₃-C₇ ou un groupe cycloalcényle en C₅-C₇, ces groupes pouvant porter encore de un à trois restes, choisis parmi les groupes alkyle en C₁-C₄, alcoxy en C₁-C₄, alkylthio en C₁-C₄, halogénoalkyle en C₁-C₄, hydroxy et halogéno ;
un hétérocycle saturé à 5 maillons, qui comprend un ou deux hétéroatomes choisis dans le groupe constitué par l'oxygène et le soufre, et qui peut porter encore de un à trois restes, choisis parmi les groupes alkyle en C₁-C₄, alcoxy en C₁-C₄, alkylthio en C₁-C₄, halogénoalkyle en C₁-C₄ ;
un hétérocycle à 6 ou 7 maillons, saturé ou comportant une ou deux insaturations, comprenant un ou deux hétéroatomes, choisis dans le groupe constitué par l'oxygène et le soufre, l'hétérocycle pouvant porter encore de un à trois restes, choisis parmi un groupe hydroxy, un atome d'halogène, un groupe alkyle en C₁-C₄, alcoxy en C₁-C₄, alkylthio en C₁-C₄, halogénoalkyle en C₁-C₄ ;
un composé hétéroaromatique à 5 maillons, contenant de un à trois hétéroatomes, choisis dans le groupe constitué par deux atomes d'azote et un atome d'oxygène ou de soufre, ce cycle pouvant porter encore de un à trois restes, choisis parmi un atome d'halogène, un groupe cyano, alkyle en C₁-C₄, alcoxy en C₁-C₄, alkylthio en C₁-C₄, halogénoa!kyle en C₁-C₄, alcényle en C₂-C₆, alcénylory en C₂-C₆, halogénoalcényle en C₂-C₆ et alcoxy(C₁-C₄)-alkyle(C₁-C₄) ;
le groupe phényle ou le groupe pyridyle, ces groupes pouvant porter encore de un à trois restes, choisis parmi un atome d'halogène, un groupe nitro, cyano, alkyle en C₁-C₄, alcoxy en C₁-C₄, alkylthio en C₁-C₄, halogénoalkyle en C₁-C₄, alcényloxy en C₃-C₆, alcinyloxy en C₃-C₆ et -NR³R⁴, où
R³ représente un atome d'hydrogène, un groupe alkyle en C₁-C₄, alcényle en C₃-C₆ ou alcinyle en C₃-C₆ et
R⁴ représente un atome d'hydrogène, un groupe alkyle en C₁-C₄, alcényle en C₃-C₆, alcinyle en C₃-C₆, acyle en C₁-C₆ ou un reste benzoyle, le cycle aromatique pouvant porter encore de un à trois restes, choisis parmi un groupe nitro, cyano, halogéno, alkyle en C₁-C₄, alcoxy en C₁-C₄, alkylthio en C₁-C₄ et halogénoalkyle en C₁-C₄,
ainsi que leurs sels et esters, formés avec des acides carboxyliques en C₁-C₁₀ et des acides inorganiques utilisables en agriculture.

2. Agent herbicide, contenant une quantité ayant une activité herbicide d'au moins un composé de formule I selon la revendication 1, et des adjuvants inertes.

3. Procédé de lutte contre des végétations indésirables, caractérisé par le fait que les végétations indésirables et/ou leur espace vital sont traités avec une quantité ayant une activité herbicide d'un dérivé de cyclohexénone de formule I selon la revendication 1.

## Revendications (Revendications pour l'(les) Etat(s) contractant(s) suivant(s): ES)

1. Procédé de préparation de cyclohexénonoximéthers de formule générale I 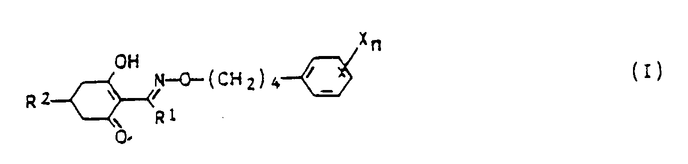 dans laquelle les variables ont les significations suivantes :
R¹ représente un groupe alkyle en C₁-C₆ ;
X représente un atome d'halogène ;
n présente une valeur de 1 à 5 ;
R² représente un groupe tétrahydropyran-3-yle, tétrahydropyran-4-yle, tétrahydrothiopyran-3-yle,
ainsi que les sels et esters du composé I, formés avec des acides carboxyliques en C₁-C₁₀ et des acides inorganiques, utilisables en agriculture, caractérisé par le fait que l'on fait réagir une cyclohexénone de formule II 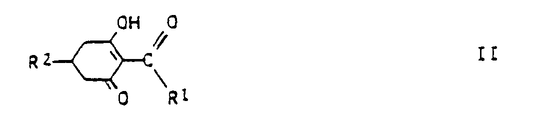 avec une hydroxylamine de formule III 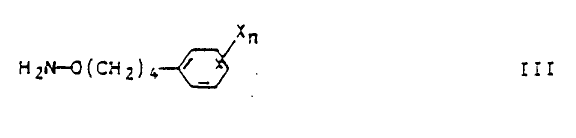 ou avec un de ses sels, éventuellement en présence d'une base, et on transforme éventuellement les produits I obtenus dans le procédé en leurs sels et esters formés avec des acides carboxyliques en C₁-C₁₀ et des acides inorganiques, utilisables en agriculture.

2. Agent herbicide, contenant une quantité ayant une activité herbicide d'au moins un composé de formule I selon la revendication 1, et des adjuvants inertes.

3. Procédé de lutte contre des végétations indésirables, caractérisé par le fait que les végétations indésirables et/ou leur espace vital sont traités avec une quantité ayant une activité herbicide d'un cyclonexénonoximéther de formule I selon la revendication 1.
